# EUROPEAN PATENT SPECIFICATION

(11) **EP 2 132 292 B1**
(45) Date of publication and mention of the grant of the patent: **08.10.2014**
(21) Application number: 08735967.5
(22) Date of filing: 08.04.2008
(51) Int. Cl.: C11D 3/386, C14C 1/08

(54) **AN ENZYMATIC TREATMENT OF SKIN AND HIDE DEGREASING**
ENZYMATISCHE BEHANDLUNG ZUR TIERHAUTENTFETTUNG
TRAITEMENT ENZYMATIQUE POUR LE DÉGRAISSAGE DES CUIRS ET PEAUX

(30) Priority: 09.04.2007 US 910702 P
(43) Date of publication of application: 16.12.2009
(73) Proprietor: Novozymes A/S, 2880 Bagsvaerd (DK)
(72) Inventor: RASMUSSEN, Lars, DK-2980 Kokkedal (DK); XU, Qing, Beijing 100080 (CN); PEDERSEN, Niels Kildegaard, DK-3480 Fredensborg (DK); ZHOU, Zhiwei, Beijing 100075 (CN)
(86) International application number: PCT/EP2008/054241
(87) International publication number: WO 2008/122640

(56) References cited:
- EP-A- 0 505 920
- WO-A-00/60063
- WO-A-94/25577
- WO-A-02/055679
- WO-A-02/066622

## Description

### FIELD OF THE INVENTION

The present invention relates to the use of lipase in degreasing skins and hides during the processing of skins and hides into leather. More specifically the lipase can be used in various steps during beam house process from pH 6-13 with or without other chemicals or surfactants.

### BACKGROUND OF THE INVENTION

Skins and hides contain regions of natural fat. However, excess fat needs to be reduced during the leather manufacturing process in order to achieve a satisfactory finish of the final leather product.

Degreasing of skins and hides is currently accomplished by use of organic solvents and surfactants.

Recently the use of lipolytic enzymes in order to improve degreasing of hides and skins has been suggested, thereby reducing or even avoiding the use of surfactants or as a substitute for organic solvents, particularly a lipase derived from *Humicola lanuginose* (EP 258068 and EP 305216) sold under the trademark GREASEX® (product of Novozymes A/S). WO 00/60063 describes a number of variants of this *Humicola lanuginose* lipase and their use in detergents, however its degreasing use in leather industry is not mentioned.

When compared to traditional methods, enzymatic degreasing processes generally improve the quality of the final leather, reduce the use of chemicals and replace chemicals which have an adverse effect on the environment.

Lipolytic enzymes hydrolyze fats into mono- and diglycerides, free fatty acids and glycerol. Lipase degreasing has been mentioned at acidic to alkaline pH conditions. However lipase takes effect in emulsified system but less effective in a one phase solution contains more than 50% of water, therefore the use of specific lipase which gives good effect in water system without adding any surfactants or keeps the surfactant use as minimum degree is a challenging problem and solving this problem will give many advantageous. Besides, if lipase can be used in both alkaline condition and neutral to acidic condition (from pH 6-13) in various steps may give other advantageous in reducing and selecting chemicals and surfactants in an optimal way.

There is an ever existing need for providing new lipases with improved degreasing properties in a variety of leather manufacturing process.

### SUMMARY OF THE INVENTION

The inventors have found that certain variants of wild type *Humicola lanuginose* lipase (these variants have been disclosed in WO 00/60063) have a particularly good degreasing performance in leather manufacture. These lipase variants can be used in degreasing in the absence of surfactants.

Accordingly, the present invention provides a process for enzymatic degreasing of skins and hides, comprising enzymatic treatment with certain variants of wild type *Humicola lanuginose* lipase in an aqueous solution under pH 6-13.

The process of the present invention improves the degreasing property and reducing the use of other chemicals and surfactants in a maximum way.

### BRIEF DISCRIPTION OF THE DRAWINGS

The present invention is further illustrated by reference to the accompanying drawing, in which:
Fig. 1 shows the fatty acids and triglycerides analysis by HPLC after the degreasing step with EUSAPON^{®} OD 4% treatment.
Fig. 2 shows the fatty acids and triglycerides analysis by HPLC after the degreasing step with Lipase 200 LU/kg wet skin treatment.
Fig. 3 shows the fatty acids and triglycerides analysis by HPLC after the degreasing step with Lipase 100 LU/kg wet skin treatment.
Fig. 4 shows the fatty acids and triglycerides analysis by HPLC after the degreasing step with Blank treatment.
   Peaks explanation in Figs 1-4:
   Peak in 5.780 min indicates palmic acid; peaks in 14-20 min indicate various triglycerides, among them 17.7-18 min is trioleate peak; peaks in 20.71-21.4 min is solvent peaks.
Fig.5: HPLC chromatogram of skin extract in example 3.
Fig.6: HPLC chromatogram of skin extract in example 4.

### DETAILED DESCRIPTION OF THE INVENTION

The present invention provides a method for the processing of hides or skins into leather, comprising enzymatic treatment of the hide or skin with a certain lipases.

### The Processing of Hides or Skins into Leather

The hides and skins are usually received in the tanneries in the form of salted or dried raw hides or skins. The processing of hides or skins into leather comprises several different process steps including the steps of soaking, unhairing, liming, deliming, bating, pickling and tanning. These steps constitute the wet processing and are performed in the beamhouse. Enzymatic treatment according to the present invention may take place at the steps of pH within 6-11 during the manufacture of leather. However, lipase degreasing of the present invention are usually employed during the wet processing, i.e., during soaking, unhairing, bating and/or pickling.

Processes for the manufacture of leather are well known to the person skilled in the art and have been described in, e.g., WO 94/06942, WO 90/12118, U.S. Patent No. 3,840,433, EP 505920, GB-A 2233665 and U.S. Patent No. 3,986,926.

### Enzymatic Degreasing

The process of the present invention may be applied to any skin or hide conventionally used for leather manufacturing. In particular, the process of the invention may be applied to ovine skins, to porcine skins, to bovine hides, or to caprine skins.

Enzymatic degreasing according to the present invention may take place at the steps of pH within 6-11 during the manufacture of leather, either as a separate step or as part of an existing leather processing step. However, the process preferably takes place during, or in between, process steps that are carried out at pH 6-13 in order to avoid unnecessary and time consuming pH adjustment.

The process of the present invention is carried out at pH 6-13. The process is preferably carried out at a pH in the range of about pH 7-11. In its most preferred embodiment the process of the invention is carried out at a pH in the range of about pH 8-11.

The process of the invention for enzymatic degreasing takes place during one or more of the subsequent steps of soaking, unhairing, liming, deliming, bating and pickling. In a leather manufacturing process, the soaking step generally takes place in the range of pH 8-9, unhairing at a pH of 9-13, deliming at a pH of 8-13 and bating at about pH 8. For the pickling step, the pH at the beginning of the step is about 8, and pH drops to around 2 at the end of this step. In a preferred embodiment, enzymatic degreasing takes place at bating step or the beginning stage of the pickling step.

In another preferred embodiment, the process of the invention for enzymatic degreasing takes place as a separate step, performed any period of time after soaking, unhairing, liming, deliming, bating or pickling has been finished. At the end of the pickling step, pH of the reaction mixture is usually in the range of pH 1-3 (around pH 2). When the process of the invention takes place during the pickling step, there will be a time slot for enzyme to take effect under pH 6-8 (e.g., 7) for 1-1.5 hours. There will be some advantageous for enzyme degreasing during pickling step since the skin structure has been opened by other chemicals or enzyme treatment in previous step, it makes easier for lipase to penetrate in the skin and take effect. On the other hand, if the lipase is added in the earlier steps, the advantageous are longer time and optimal pH for lipase to take effect although the penetration of lipase is worse than added in the pickled step.

The process of the invention may be carried out at temperatures normally employed in leather manufacturing processes, i.e., in the range of from about 15°C to about 65°C, or even up to about 75°C. Dependent *inter alia* on the hide or skin in question the temperature preferably is kept in the range of from about 20°C to about 40°C (in particular when applied to ovine hides), or in the range of from about 30°C to about 40°C (in particular when applied to bovine hides).

### Enzymatic Treatment of Skins and Hides

In a process according to the present invention for enzymatic degreasing of skins and hides, the skin or hide is treated with a lipase in an aqueous reaction medium, in order to hydrolyze fats present in the skin or hide. '

The degreasing with the lipase of the present invention works well in the absent of surfactants. When surfactant is used, the surfactant preferably is an anionic, a non-ionic, or an amphoteric type surfactant, or a mixture thereof. Moreover, organic solvents may be present during the lipolytic treatment, but organic solvents are not needed in a process of the invention. Therefore, out of environmental concern, the reaction mixture should be kept free of organic solvents.

The reaction time greatly depends on the process requirement, basically the lipases works well under most of the process conditions in the beam house. For practical reasons a reaction time in the range of 30 minutes to 24 hours is contemplated. Preferably the reaction time is in the range of 0.5-16 hours, more preferred 0.5-4 hours, most preferred 0.5-2 hours.

When hydrolysis takes place, hydrolysis products are formed. These reaction products should be removed from hides and skins.

Hydrolysis products may be removed by separating hides and skins from the aqueous reaction medium. Preferably the hides and skins are subsequently washed repeatedly with water. In case of the process has a higher pH (e.g., pH>11), the saponification will automatically happens with the fatty acids formed by lipase and therefore to produce soaps helping emulsification of the fat and fatty acids without adding more external surfactants. Therefore the use of such lipase will reduce the use of surfactants in a maximum way.

The surface active agent used in an aqueous mixture for removal of the hydrolysis products may be any conventional surfactants. However, anionic, non-ionic and amphoteric type surfactants are preferred, either as separate surfactants or in mixture.

### Lipase

The reference lipase used in this invention is the wild type *Humicola lanuginose* lipase derived from *Humicola lanuginose* strain DSM 4109. It is described in EP 258068 and EP 305216 and has the amino acid sequence shown in position 1-269 of SEQ ID NO: 2 of U.S. Patent No. 5,869,438. In this specification, the reference lipase is shown as amino acids 1-269 of SEQ ID NO: 1 of the present invention.

The variants of this *Humicola lanuginose* lipase and it use in detergent have already been mentioned in WO 00/60063.

The variant lipases of course have lipase activity and show good effect in degreasing in leather manufacture. Lipases comprise conservative substitutions, insertions, deletions, N-terminal extensions, and/or C-terminal extensions, as well as lipase fragments as compared to the sequence of amino acids 1-269 of SEQ ID NO: 1 can be prepared from this molecule by any method known in the art, such as site-directed mutagenesis, random mutagenesis, consensus derivation processes (EP 897985), and gene shuffling (WO 95/22625, WO 96/00343), etc.

The amino acid changes allowed for the variant lipase are of a minor nature, that is conservative amino acid substitutions or insertions that do not significantly affect the folding and/or activity of the protein, preferably a small number of such substitutions or insertions; small deletions; small amino- or carboxyl-terminal extensions, etc. In the above context, the term "small" independently designates a number of up to 25 amino acid residues. In preferred embodiments, the term "small" independently designates up to 24, 23, 22, 21, or up to 20 amino acid residues. In additional preferred embodiments, the term "small" independently designates up to 19, 18, 17, 16, 15, 14, 13, 12, 11, or up to 10 amino acid residues. In further preferred embodiments, the term "small" independently designates up to 9, 8, 7, 6, 5, 4, 3, 2, or up to 1 amino acid residue.

The variant lipase of the invention has been derived from a parental lipase of amino acids 1-269 of SEQ ID NO: 1 by double-substitution of T231 R and N233R.

The nomenclature used herein for defining mutations is essentially as described in WO 92/05249. Thus, T231R indicates a substitution of T in position 231 with R.

The lipase of the invention may be applied in concentrations conventionally employed in degreasing processes. The lipase may be added in an amount of from 10 to 600 KLU per kg of hide or skin, preferably of from 50 to 400 KLU per kg of hide or skin, more preferably of from 100 to 300 KLU per kg of hide or skin.

### Leather Degreasing Composition

The lipase of the present invention can be used together with surfactant in degreasing. Additionally, the composition may comprise another enzyme and other components conventionally used in leather industry.

The leather degreasing composition according to the invention can be in liquid, paste, gels, bars or granular forms.

The lipase of the invention, or optionally another enzyme incorporated in the leather degreasing composition, is normally incorporated in the composition at a level from 0.00001% to 3% of enzyme protein by weight of the composition, preferably at a level from 0.001 % to 2% of enzyme protein by weight of the composition, more preferably at a level from 0.01 % to 1% of enzyme protein by weight of the composition, even more preferably at a level from 0.1% to 1% of enzyme protein by weight of the composition.

### Surfactant system

The surfactant system may comprise nonionic, anionic, cationic, ampholytic, and/or zwitterionic surfactants. As described above, the lipase variants of the invention are particularly suited for leather degreasing comprising of a combination of anionic and nonionic surfactant with 0-40% by weight of anionic surfactant and 60-100% by weight of nonionic, particularly 0-30% of anionic surfactant and 70-100% nonionic. As further described, some preferred lipases of the invention are also suited for leather degreasing comprising 20-30% anionic and 70-80% non-ionic surfactant.

The surfactant is typically present at a level from 0.1 % to 60% by weight of composition, e.g., 1% to 40%, particularly 3% to 30%, preferably from 10-30%, more preferably from about 12% to about 25% by weight. Some examples of surfactants are described below.

### Anionic surfactants

Preferred anionic surfactants include alkyl sulfate, alkyl ethoxy sulfate, linear alkyl benzene sulfonate and mixtures of these.

The alkyl sulfate surfactants are water soluble salts or acids of the formula ROSO₃M wherein R preferably is a C₁₀-C₂₄ hydrocarbyl, preferably an alkyl or hydroxyalkyl having a C₁₀-C₂₀ alkyl component, more preferably a C₁₂-C₁₈ alkyl or hydroxyalkyl, and M is H or a cation, e.g., an alkali metal cation (e.g., sodium, potassium, lithium), or ammonium or substituted ammonium.

Alkylbenzene sulfonates are suitable, especially linear (straight-chain) alkyl benzene sulfonates (LAS) wherein the alkyl group preferably contains from 10 to 18 carbon atoms.

Suitable anionic surfactants include alkyl alkoxylated sulfates which are water soluble salts or acids of the formula RO(A)ₘSO₃M wherein R is an unsubstituted C₁₀-C-₂₄ alkyl or hydroxyalkyl group having a C₁₀-C₂₄ alkyl component, preferably a C₁₂-C₂₀ alkyl or hydroxyalkyl, more preferably C₁₂-C₁₈ alkyl or hydroxyalkyl, A is an ethoxy or propoxy unit, m is greater than zero, typically between about 0.5 and about 6, more preferably between about 0.5 and about 3, and M is H or a cation which can be, for example, a metal cation (e.g., sodium, potassium, lithium, calcium, magnesium, etc.), ammonium or substituted-ammonium cation. Alkyl ethoxylated sulfates as well as alkyl propoxylated sulfates are contemplated herein. Specific examples of substituted ammonium cations include methyl-, dimethyl, trimethyl-ammonium cations and quaternary ammonium cations such as tetramethyl-ammonium and dimethyl piperdinium cations and those derived from alkylamines such as ethylamine, diethylamine, triethylamine, mixtures thereof, and the like.

Other anionic surfactants include salts (including, for example, sodium, potassium, ammonium, and substituted ammonium salts such as mono-, di- and triethanolamine salts) of soap, C₈-C₂₂ primary or secondary alkanesulfonates, C₈-C₂₄ olefinsulfonates, sulfonated polycarboxylic acids prepared by sulfonation of the pyrolyzed product of alkaline earth metal citrates.

### Nonionic surfactant

The surfactant may comprise polyalkylene oxide (e.g., polyethylene oxide) condensates of alkyl phenols. The alkyl group may contain from about 6 to about 14 carbon atoms, in a straight chain or branched-chain. The ethylene oxide may be present in an amount equal to from about 2 to about 25 moles per mole of alkyl phenol.

The surfactant may also comprise condensation products of primary and secondary aliphatic alcohols with about 1 to about 25 moles of ethylene oxide. The alkyl chain of the aliphatic alcohol can either be straight or branched, and generally contains from about 8 to about 22 carbon atoms.

Further, the nonionic surfactant may comprise polyethylene oxide condensates of alkyl phenols, condensation products of primary and secondary aliphatic alcohols with from about 1 to about 25 moles of ethylene oxide, alkylpolysaccharides, and mixtures hereof. Most preferred are C₈-C₁₄ alkyl phenol ethoxylates having from 3 to 15 ethoxy groups and C₈-C₁₈ alcohol ethoxylates (preferably C₁₀ avg.) having from 2 to 10 ethoxy groups, and mixtures thereof.

Preferred nonionic surfactants are alcohol ethoxylate, alcohol phenol ethoxylate, polyhydroxy fatty acid amide, alkyl polyglucoside and mixtures of these. The commercially available example of nonionic surfactant is EUSAPON@ OD, Lutensol® ON60 or Neodol® 25-7.

### Lipolytic Activity

The lipolytic activity may be determined using tributyrine as substrate. This method is based on the hydrolysis of tributyrine by the enzyme, and the alkali consumption is registered as a function of time.

One Lipase Unit (LU) is defined as the amount of enzyme which, under standard conditions (i.e., at 30°C; pH 7.0; with Gum Arabic as emulsifier and tributyrine as substrate) liberates 1 micro mol titritable butyric acid per minute.

### EXAMPLES

The invention is further illustrated with reference to the following examples, which are not intended to be in any way limiting to the scope of the invention as claimed.

### Example 1: Enzymatic Degreasing of sheep skin

This example demonstrated the process of the invention as applied to pickled England domestic sheep skin. The skin was subjected to lipase treatment with a lipase variant of two substitutions T231 R+N233R in SEQ ID NO: 1 (obtained according to WO 00/60063).

**Fat extraction and fat content test:** After finishing the degreasing trial, the skin pieces were collected and rinsed by water for 2 min, then dried in an oven at 40°C overnight. The dried skins were weighted and extracted by CHCl₃ in SOXTECH™ equipment (available from FOSS Company), soaked for 50 min, extracted for 180 min under 120°C, solvent recover time was 50 min. The fat content was calculated as the weight loss after the extraction.

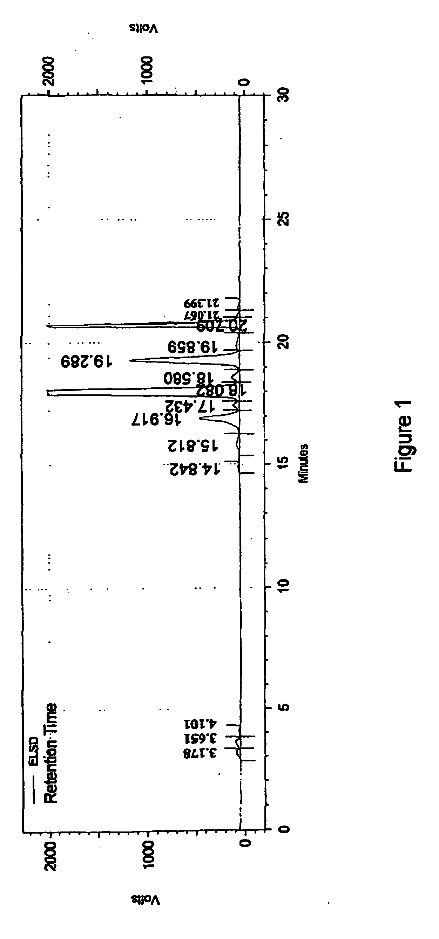

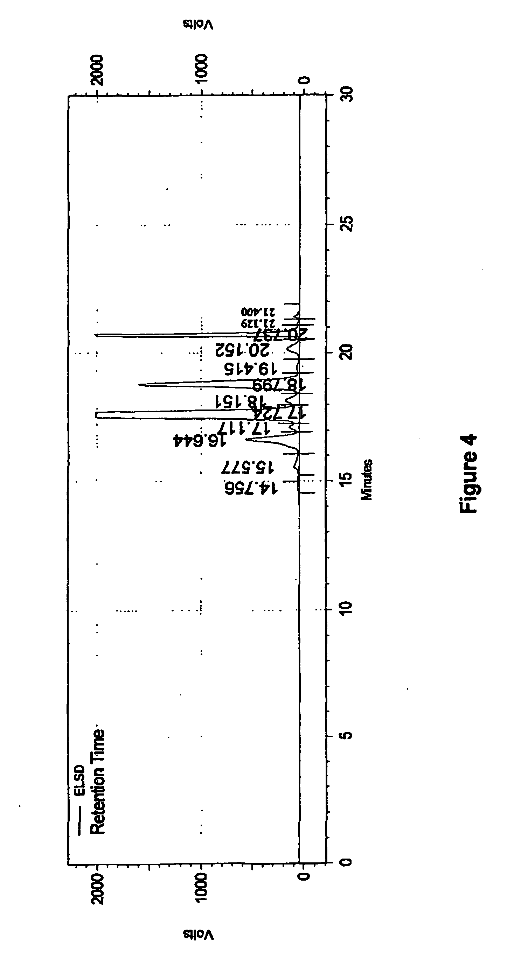

**Fatty acids and triglycerides analysis by HPLC:** Waters 2690 separations module including a four solvent gradient system and LiChrospher 100RP-8 endcapped (5um) column (Merck) was used. A tri-gradient solvent elution including HCN, 0.1% AcOH in water and CH₂Cl₂/HCN was used for separation FA (fatty acids), and TG (Triglycerides). Oleic acid, palmic acid, stearic acid and trioleate standards are used from 0.2-1.0 mg/ml to calculate the formation of fatty acids and residue TG in the CHCl₃ extracts. Figures 1-4 gave the HPLC charts.

Residue trioleate: trioleate is a triglyceride containing three identical oleic acids in each of the ester bonds. Since it is the most widely existed TG in animal skin, it can be used as an index to see the degreasing degree.

Palmic acid: it is one of the popular fatty acid type exist in animal skin, since the oleic acid has lower melting point, it can go into the wash liquor during degreasing and the following process, therefore most of the remaining fatty acids left in sheep skin are high melting point fatty acids, and palmic acid is one of them. It is measured to indicate the formation of fatty acids in the sheep skin.

**Degreasing steps:** Raw material-pickled England domestic sheep skin, was cut into 1*1 cm from low fat positions and 0.5*0.5 cm pieces from high fat positions (shoulders, necks & buttocks), total 40 g mixture each LOM beaker, add four steel ball in LOM beaker to increase mechanical strength. Degreasing conditions was shown in Table 1.

**Table 1**

| Process | Leather (g) | Buffer (ml) | pH | Temperature (°C) | Time (min) |
|---|---|---|---|---|---|
| Degreasing | 40 g | 50 ml | 7 | 30 | 60 |
| Saponification | | | 12 | 30 | 60 |
| Drain | | | | | |
| Wash the skin pieces in running water | | | | | 2 |

| | | | | | |
|---|---|---|---|---|---|
| Buffer contains 8 wt% NaCl and 4 wt% NaHCO₃. | | | | | |

The example was carried out as five groups of trial running in parallel (see group 1-5 of Table 2). The surfactant or lipases were added in the buffer according to Table 2 in degreasing step for each of the comparison trials. Figures 1-4 showed the HPLC charts of experiments of groups 1, 2, 3 and 5 respectively.

Among them, EUSAPON^{®} OD 4% was used in an industrial standard degreasing method. Blank was only buffer. Untreated was the original pickled skin without any degreasing treatment used as a comparison.

**Table 2**

| Group NO | Treatment | Fat % (CHCl₃ extraction on degreased skin) | Residue Trioleate (mg/ml by HPLC) | Palmic acid residue (mg/ml by HPLC) |
|---|---|---|---|---|
| 1 | EUSAPON^{®} OD 4% | 14.5 | 15.2 | 0 |
| 2 | Lipase 200 KLU/kg wet skin | 6.7 | Can not be detected | 0 |
| 3 | Lipase 100 KLU/kg wet skin | 8.2 | <0.2 | 16.4 |
| 4 | EUSAPON^{®} OD 4%(repeat) | 14.1 | 13.6 | 0 |
| 5 | Blank | 15.5 | 16.4 | 0 |
| 6 | Untreated | 19.0 | 17.8 | 0 |

The fat % was calculated according to weight loss in CHCl₃ extraction, it can be either the FA or TG or DG (diglycerides) or MG (monoglycerides) or their combinations. The fat % remained in the skin after lipase treatment was 6.7% (group 2) or 8.2% (group 3), while the fat % remained after surfactant Eusapon OD 4% treatment was more than 14%. It indicates that the degreasing effect of the lipase of the present invention was much better than that of the surfactant.

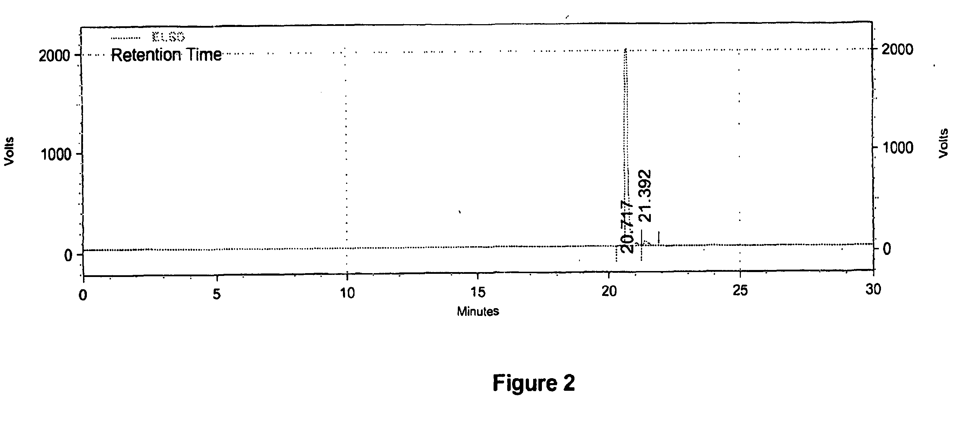

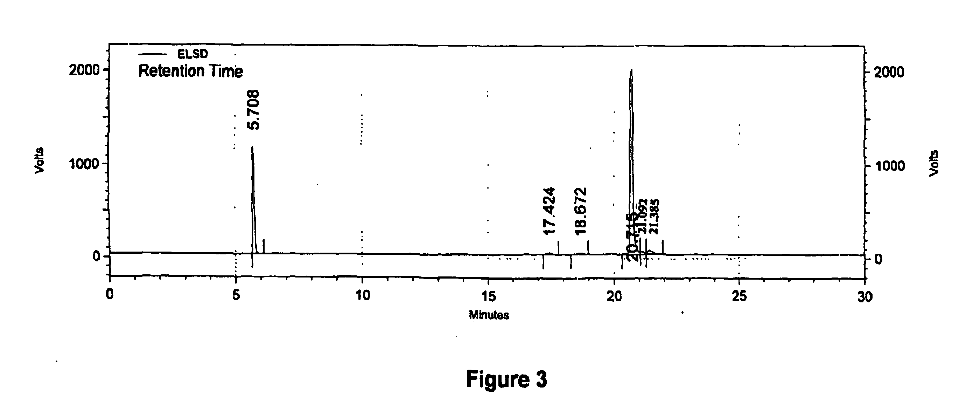

Residue trioleate and palmic acid content in the extracts were calculated according to the standard curves measured by HPLC. The HPLC peaks for TGs and trioleate were not shown in the lipase treatment samples (Figs. 2 and 3) while the residue trioleate in the extracts treated by Eusapon was as high as 15.2 mg/ml (Table 2). These results meant 100-200 KLU/kg lipase completely degraded all TGs in sheep skin under test condition, thus the measured fat % in lipase treated samples might be fatty acids mixtures or protein hydrolysates or soap.

Further, it can be seen from the HPLC chart, there was no fatty acid peaks for 200 KLU lipase/kg skin treatment, which means the fatty acids might have been transferred into soaps by saponification, it may still left inside the skin or has been transferred out of the skin. While using 100 KLU lipase/kg skin, since the speed to form fatty acids was slower than that using 200 KLU lipase, the saponification was not completed, there is only one fatty acid peaks can be detected, the peak position is the same as palmic acid and oleic acid retention time, since oleic acid has lower melting points, it transferred into liquid and be washed easily than palmic acid. Therefore it was suspected most of the fatty acid detected by this peak was palmic acid which was 16.4 mg/ml (Table 2).

### Example 2. Comparison of lipases on pickled New Zealand sheep skin

Sheep skins are prepared in the same way as example 1. The trial is carried out under the following condition.

**Degreasing steps:** Raw material-pickled New Zealand sheep skin was prepared as example 1. Degreasing conditions was shown under table 3.

**Table 3**

| Process | Leather (g) | Buffer (ml) | pH | Temperature (°C) | Time (min) |
|---|---|---|---|---|---|
| Degreasing | 40 g | 50 ml | 7 | 30 | 60 |
| Drain | | | | | |
| Wash the skin pieces in running water | | | | | 2 |

| | | | | | |
|---|---|---|---|---|---|
| Buffer contains 8 wt% NaCl and 4 wt% NaHCO₃. | | | | | |

The example was carried out as four groups of trial running. Two lipases were added in the buffer according to table 3 in degreasing step for each of the comparison trials. Blank was only buffer. Untreated was the original pickled skin without any degreasing treatment used as a comparison.

**Table 4**

| Group No. | Treatment | Wet weight skin (g) | Dried weight skin | Fat % |
|---|---|---|---|---|
| 1 | Blank | 40.47 | 15.898 | 17.9 |
| 2 | Reference Lipase 200 KLU/kg wet skin | 40.62 | 17.157 | 17.4 |
| 3 | Lipase 200 KLU/kg wet skin | 40.63 | 17.007 | 13.6 |
| 4 | Untreated | 40.39 | 18.303 | 18.8 |

Reference lipase used in Group 2: Parent *Humicola lanuginose* lipase as SEQ ID NO: 1 of the present application.

Lipase used in Group 3: lipase variant with two substitutions T231 R+N233R in SEQ ID NO: 1.

From the result in table 4, it can be seen when there are no surfactants in the system, lipases act differently in degreasing. Fat residue of variant lipase in group 3 is 13.6%, which it is much lower than that of Reference lipase which is almost the same as blank (17.4 vs. 17.9).

### Example 3: Surfactant and Lipase synergies under liming condition

This example demonstrated the degreasing process as applied to limed sheep skin (available from Shanghai Oujiayu Trading Co., Ltd. PRC). The lipase used in this example and example 4 is a lipase variant of two substitutions T231R+N233R in SEQ ID NO: 1 (obtained according to WO 00/60063).

Degreasing step: Raw material-limed sheep skin was prepared as example 1. Degreasing conditions was shown under Table 5. The buffer used in this example is water to mimic liming condition.

**Table 5**

| Process | Leather (g) | Buffer (ml) | pH | Temperature (°C) | Time (min) |
|---|---|---|---|---|---|
| Degreasing | 50 g | 100 ml | 12 | 30 | 60 |
| Drain | | | | | |
| Wash the skin pieces in running water | | | | | 2 |

The example was carried out as eight groups of trial running. Lipase and/or surfactant EUSAPON@ OD were added in the buffer according to Table 6 by weight of skin in degreasing step for each of the comparison trials. Blank was only buffer.

**Table 6**

| Group No. | Treatment | Skin | Buffer | Lipase* | Eusapon OD** |
|---|---|---|---|---|---|
| 1 | Blank | 50 g | 100 ml | - | - |
| 2 | 0.5% T1 | 50 g | 99 ml | - | 1 ml |
| 3 | 1% T1 | 50 g | 98 ml | - | 2 ml |
| 4 | 0.05% L | 50 g | 99 ml | 1 ml | - |
| 5 | 0.1% L | 50 g | 98 ml | 2 ml | - |
| 6 | 0.1%L+0.5%T1 | 50 g | 97 ml | 2 ml | 1 ml |
| 7 | 0.1%L+1%T1 | 50 g | 96 ml | 2 ml | 2 ml |
| 8 | 0.1%L+2%T1 | 50 g | 94 ml | 2 ml | 4 ml |

| | | | | | |
|---|---|---|---|---|---|
| T1 represents surfactant EUSAPON^{®} OD, L represents Lipase. * The concentration of lipase is 25 mg/ml. ** The concentration of Eusapon OD is 250 mg/ml. | | | | | |

Fat extraction test was conducted according to the method in example 1, except that hexane was used in this example instead of CHCl₃.

HPLC analysis was used to analyze residual fat composition, but only representative peaks were used to quantify triglyceride or free fat acid content.

### Results

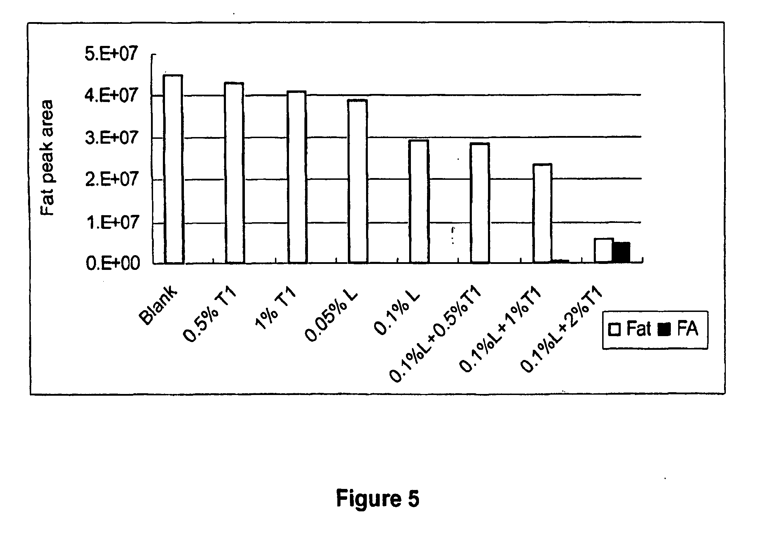

It is clear from Figure 5 that in the skin no significant amount of fat has been removed by T1 alone at the dose of 0.5% and 1%, respectively. A dose-dependent fat reduction and liberation of FAs (FA in Figure 5 represents fatty acids) by EUSAPON® OD can be obtained when used together with 0.1% Lipase, and the reduction became manefested when 2% EUSAPON® OD had been dosed with only small amount residual FAs seen on the HPLC chromatograph. The results indicate obvious synergistic degreasing effects between Lipase and surfactant especially at higher dosage.

### Example 4: Surfactant and Lipase synergies under deliming condition

Degreasing steps: Raw material-limed sheep skin was prepared as example 1. Degreasing conditions were shown under Table 7. The buffer used in this example is 1.5% (NH₄)₂SO₄ to mimic deliming condition.

**Table 7**

| Process | Leather (g) | Buffer (ml) | pH | Temperature (°C) | Time (min) |
|---|---|---|---|---|---|
| Degreasing | 50 g | 100 ml | 8 | 30 | 120 |
| Drain | | | | | |
| Wash the skin pieces in running water | | | | | 2 |

The example was carried out as eight groups of trial running. Lipase and/or surfactant were added in the buffer according to Table 8 by weight of skin in degreasing step for each of the comparison trials. Blank was only buffer.

The nonionic surfactant were Lutensol® ON60 and Neodol® 25-7, while the anionic surfactant was short-chain LAS with an average hydrocarbon chain length of 11, with 25% LAS, 25% Neodol® 25-7 and 50% Lutensol® ON60.

**Table 8**

| Group No. | Treatment | Skin | 1.5% (NH₄)₂SO₄ | Lipase* | T2** |
|---|---|---|---|---|---|
| 1 | Blank | 50 g | 100 ml | - | - |
| 2 | 5%T2 | 50 g | 99 ml | - | 10 ml |
| 3 | 10%T2 | 50 g | 90 ml | - | 20 ml |
| 4 | 0.1%L | 50 g | 80 ml | 1 ml | - |
| 5 | 0.1%L+0.5%T2 | 50 g | 98 ml | 1 ml | 1 ml |
| 6 | 0.1%L+1%T2 | 50 g | 97 ml | 1 ml | 2 ml |
| 7 | 0.1%L+2%T2 | 50 g | 95 ml | 1 ml | 4 ml |
| 8 | 0.1 %L+5%T2 | 50 g | 89 ml | 1 ml | 10 ml |

| | | | | | |
|---|---|---|---|---|---|
| T2 represents surfactant, a mixture of 25% LAS, 25% Neodol® 25-7 and 50% Lutensol® ON60; L represents Lipase. * The concentration of lipase is 50 mg/ml. ** The concentration of T2 is 250 mg/ml. Fat extraction test and HPLC analysis were conducted as example 3. | | | | | |

### Results

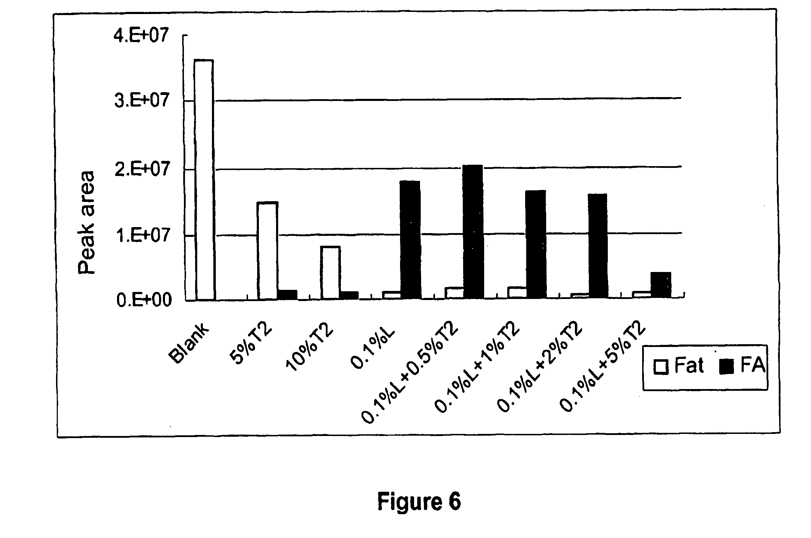

Figure 6 indicates that there is a synergistic effect from T2 and lipase on fat hydrolysis since T2 and Lipase together produced more FA and less TG than either of them used alone. At higher T2 offer, better FA removal becomes obvious. T2 may have very good FA removing capability. As shown in Fig. 6, 0.1 % Lipase combined with 5% T2 produced lower fat content and a little bit higher FA than 10% T2 alone illustrating synergistic degreasing effect between them.

### SEQUENCE LISTING

<110> Novozymes A/S
<120> An enzymatic treatment of skin and hide degreasing
<130> 11216.000
<160> 1
<170> PatentIn version 3.4
<210> 1
   <211> 269
   <212> PRT
   <213> Humicola lanuginosa
<220>
   <221> mat_peptide
   <222> (1)..(269)
<400> 1

## Claims

1. A process for enzymatic degreasing of skins or hides, which process comprises treatment of the skin or hide with a lipase variant which has been derived from a parental lipase of amino acids 1-269 of SEQ ID NO: 1 by the double-substitution T231R and N233R, and wherein treatment with the lipase takes place during one or more of the steps of soaking, unhairing, deliming and pickling.

2. The process according to claim 1, wherein the process is applied to ovine skins, porcine skins, bovine skins, or caprine skins.

3. The process according to any of claims 1-2, wherein the lipase treatment takes place in an aqueous medium optionally in the presence of a surfactant.

4. The process according to any of claims 1-3, wherein the process takes place as a separate step after soaking, unhairing, liming, deliming, bating and pickling.

5. The process according to any of claims 1-4, wherein the lipase is added in an amount of from 10 to 600 KLU per kg of hide or skin.

6. The process according to claim 5, wherein the lipase is added in an amount of from 50 to 400 KLU per kg of hide or skin.

7. The process according to any of claims 1-4, wherein the process is carried out at a pH in the range of from 6 to 13.

8. The process according to claim 7, wherein the process is carried out at a pH in the range of from 7 to 11.

9. The process according to any of claims 1-7, wherein the process is carried out at a temperature in the range of from 15 to 65°C.

10. The process according to claim 9, wherein the process is carried out at a temperature in the range of from 20 to 40°C.

## Patentansprüche

1. Verfahren zum enzymatischen Entfetten von Häuten und Fellen, wobei das Verfahren die Behandlung der Haut oder des Fells mit einer Lipasevariante umfasst, die aus einer parentalen Lipase von Aminosäuren 1-269 von SEQ ID NO: 1 durch die Doppelsubstitution T231R und N233R abgeleitet worden ist, und wobei die Behandlung mit der Lipase während einem oder mehreren der Schritte des Einweichens, Enthaarens, Entkalkens und Pickeln stattfindet.

2. Verfahren nach Anspruch 1, wobei das Verfahren auf Schafhäute, Schweinehäute, Rinderhäute oder Ziegenhäute angewandt wird.

3. Verfahren nach einem beliebigen der Ansprüche 1-2, wobei die Lipasebehandlung in einem wässrigen Medium, gegebenenfalls in der Gegenwart eines oberflächenaktiven Mittels, stattfindet.

4. Verfahren nach einem beliebigen der Ansprüche 1-3, wobei das Verfahren als ein separater Schritt nach dem Einweichen, Enthaaren, Kalken, Entkalken, Beizen und Pickeln stattfindet.

5. Verfahren nach einem beliebigen der Ansprüche 1-4, wobei die Lipase in einer Menge von 10 bis 600 KLU pro kg Haut oder Fell hinzugefügt wird.

6. Verfahren nach Anspruch 5, wobei die Lipase in einer Menge von 50 bis 400 KLU pro kg Haut oder Fell hinzugefügt wird.

7. Verfahren nach einem beliebigen der Ansprüche 1-4, wobei das Verfahren bei einem pH im Bereich von 6 bis 13 durchgeführt wird.

8. Verfahren nach Anspruch 7, wobei das Verfahren bei einem pH im Bereich von 7 bis 11 durchgeführt wird.

9. Verfahren nach einem beliebigen der Ansprüche 1-7, wobei das Verfahren bei einer Temperatur im Bereich von 15 bis 65 °C durchgeführt wird.

10. Verfahren nach Anspruch 9, wobei das Verfahren bei einer Temperatur im Bereich von 20 bis 40 °C durchgeführt wird.

## Revendications

1. Procédé pour le dégraissage enzymatique de peaux ou de cuirs, ledit procédé comprenant le traitement de la peau ou du cuir avec un variant de lipase qui a été dérivé à partir d'une lipase parentale d'acides aminés 1 à 269 de SEQ ID NO: 1 par la double substitution T231R et N233R, et dans lequel le traitement avec la lipase a lieu au cours d'une ou plusieurs des étapes de trempage, ébourrage, déchaulage et décapage.

2. Procédé selon la revendication 1, ledit procédé étant appliqué à des peaux d'ovins, à des peaux de porcins, à des peaux de bovins, ou à des peaux de caprins.

3. Procédé selon l'une quelconque des revendications 1 à 2, dans lequel le traitement à la lipase a lieu dans un milieu aqueux, optionnellement en présence d'un agent tensioactif.

4. Procédé selon l'une quelconque des revendications 1 à 3, ledit procédé se déroulant comme une étape distincte après trempage, ébourrage, chaulage, déchaulage, confit et décapage.

5. Procédé selon l'une quelconque des revendications 1 à 4, dans lequel la lipase est ajoutée en une quantité de 10 à 600 KLU par kg de cuir ou de peau.

6. Procédé selon la revendication 5, dans lequel la lipase est ajoutée en une quantité de 50 à 400 KLU par kg de cuir ou de peau.

7. Procédé selon l'une quelconque des revendications revendication 1 à 4, ledit procédé étant réalisé à un pH dans la gamme de 6 à 13.

8. Procédé selon la revendication 7, ledit procédé étant réalisé à un pH dans la gamme de 7 à 11.

9. Procédé selon l'une quelconque des revendications 1 à 7, ledit procédé étant réalisé à une température dans la gamme de 15 à 65°C.

10. Procédé selon la revendication 9, ledit procédé étant réalisé à une température dans la gamme de 20 à 40°C.
